# FASCICULE DE BREVET EUROPEEN

(11) **EP 3 749 227 B1**
(45) Date de publication et mention de la délivrance du brevet: **05.04.2023**
(21) Numéro de dépôt: 19710451.6
(22) Date de dépôt: 06.02.2019
(51) Int. Cl.: A61B 17/17, A61B 17/72

(54) **DISPOSITIF D'ARTHRODÈSE AMÉLIORÉ**
VERBESSERTE ARTHRODESEVORRICHTUNG
IMPROVED ARTHRODESIS DEVICE

(30) Priorité: 07.02.2018 FR 1851024
(43) Date de publication de la demande: 16.12.2020
(60) Demande divisionnaire: 23152050.3
(73) Titulaire: In2Bones, 69130 Ecully (FR)
(72) Inventeur: KEARNS, Stephen, Barna, Galway (IE)
(74) Mandataire: Weber, Jean-François
(86) Numéro de dépôt international: PCT/FR2019/050263
(87) Numéro de publication internationale: WO 2019/155160

(56) Documents cités:
- WO-A1-2006/091460
- WO-A1-2011/072249
- US-A1- 2012 109 217
- US-A1- 2012 130 370

## Description

### DOMAINE TECHNIQUE

La présente invention se rapporte au domaine technique général des dispositifs destinés à être utilisés pour réaliser une arthrodèse et, plus spécifiquement, pour réaliser une arthrodèse de l'articulation d'une cheville, en particulier dans le cadre d'un traitement orthopédique.

L'invention concerne plus précisément un dispositif d'arthrodèse de cheville comprenant un clou destiné à être implanté dans un tibia, un talus et un calcanéus d'un patient, ledit clou comprenant une partie proximale qui s'étend généralement selon une première direction d'extension longitudinale, et une partie distale qui prolonge ladite partie proximale et s'étend généralement selon une deuxième direction d'extension longitudinale, ladite partie distale étant pourvue d'un premier orifice de fixation.

### TECHNIQUE ANTERIEURE

Afin de traiter certaines pathologies osseuses de la cheville, telle que l'arthrose, entraînant une dégradation ou une disparition du cartilage articulaire, il est connu de procéder à une arthrodèse de l'articulation de la cheville concernée, c'est-à-dire à une intervention chirurgicale destinée à supprimer toute ou partie de la mobilité de l'articulation de la cheville en provoquant une ostéosynthèse (ou « fusion osseuse ») des corps osseux en présence.

Pour réaliser de telles interventions, il est connu d'utiliser un dispositif d'arthrodèse comprenant un clou d'arthrodèse, destiné à être implanté au niveau de l'articulation de la cheville. Ce clou est généralement introduit dans des logements ménagés successivement à travers les différents corps osseux formant l'articulation concernée, puis fixés à ces derniers, de sorte à immobiliser l'articulation de la cheville et à favoriser une fusion osseuse de ces corps osseux. En général, la mise en œuvre d'un tel clou d'arthrodèse comprend, après l'implantation du clou et avant la fixation de ce dernier, une opération de compression des corps osseux de l'articulation de la cheville, destinée à rapprocher ces derniers, et en particulier le calcanéum et le tibia, afin de favoriser notamment une bonne fusion osseuse de ces derniers. Le document WO 2011/072249 A1 définit le préambule de la revendication 1.

Si de tels dispositifs d'arthrodèse connus donnent généralement satisfaction et ont permis à ce jour une nette amélioration de la prise en charge de patients souffrants de pathologies osseuses avancées de la cheville, ils n'en restent cependant pas moins perfectibles. En effet, il a été en particulier observé que le clou d'arthrodèse de tels dispositifs, une fois implanté dans le corps du patient, peut dans certains cas présenter un manque de stabilité à l'usage et conduire à un maintien insuffisant des corps osseux de l'articulation concernée. Il en résulte alors un risque d'ostéosynthèse imparfaite des corps osseux de la cheville.

### EXPOSE DE L'INVENTION

Les objets assignés à la présente invention visent en conséquence à remédier aux inconvénients susmentionnés et à proposer un nouveau dispositif d'arthrodèse comprenant un clou qui, par sa configuration, présente une fois implanté dans l'articulation de la cheville d'un patient, une excellente stabilité tout en permettant un maintien amélioré des corps osseux de la cheville pour favoriser la fusion osseuse de ces derniers.

Un autre objet de l'invention vise à proposer un nouveau dispositif d'arthrodèse comprenant un clou d'arthrodèse particulièrement adapté à l'anatomie de l'articulation de la cheville.

Un autre objet de l'invention vise à proposer un nouveau dispositif d'arthrodèse comprenant un clou qui peut être fixé de manière particulièrement efficace dans les corps osseux de l'articulation de la cheville.

Un autre objet de l'invention vise à proposer un nouveau dispositif d'arthrodèse comprenant un clou d'arthrodèse de conception simple et peu onéreux à fabriquer.

Un autre objet de l'invention vise à proposer un nouveau dispositif d'arthrodèse permettant d'effectuer une compression efficace de l'articulation de la cheville.

Un autre objet de l'invention vise à proposer un nouveau dispositif d'arthrodèse permettant de traiter une pathologie osseuse de la cheville du patient de façon particulièrement efficace et rapide.

Les objets assignés à l'invention sont atteints à l'aide d'un dispositif d'arthrodèse de cheville selon la revendication 1.

### DESCRIPTIF SOMMAIRE DES DESSINS

D'autres particularités et avantages de l'invention apparaîtront et ressortiront plus en détails à la lecture de la description faite ci-après, en référence aux dessins annexés, donnés uniquement à titre d'exemple illustratif et non limitatif, dans lesquels :
- la figure 1 illustre, selon une vue latérale, un mode de réalisation préférentiel du clou d'un dispositif d'arthrodèse conforme à l'invention, lequel clou comprend une partie proximale qui s'étend généralement selon une première direction d'extension longitudinale et une partie distale qui prolonge ladite partie proximale et s'étend généralement selon une deuxième direction d'extension longitudinale, laquelle est sécante à ladite première direction d'extension ;
- les figures 2 et 3 illustrent respectivement selon des vues antérieure (figure 2) et postérieure (figure 3), le clou de la figure 1 ;
- la figure 4 illustre, en vue de dessous, le clou des figures 1 à 3, la première direction d'extension de la partie proximale du clou étant orthogonale au plan de la figure ;
- les figures 5 et 6 illustrent, respectivement selon une vue latérale (figure 5) et selon une vue médiale en perspective (figure 6), le clou des figures précédentes, implanté dans une articulation d'une cheville droite d'un patient. Sur ces figures, le clou en question est fixé aux corps osseux de l'articulation de la cheville à l'aide d'une pluralité de moyens de fixation indépendants du clou ;
- Les figures 7 et 8 illustrent le clou des figures précédentes, implanté dans une articulation d'une cheville droite d'un patient, en lien avec un instrument externe de mise en place dudit clou ;
- La figure 9 illustre, selon une vue en perspective, un détail de l'instrument de la figure 7 et de la partie distale du clou des figures 1 à 3.

### MEILLEURE MANIERE DE REALISER L'INVENTION

L'invention concerne un dispositif d'arthrodèse, conçu pour être mis en oeuvre dans le cadre d'une arthrodèse de l'articulation d'une cheville d'un patient, en particulier lorsque cette dernière se trouve dans un état de dégradation trop avancé pour que d'autres interventions chirurgicales moins sévères puissent être techniquement envisagées. De préférence, le patient concerné par le dispositif d'arthrodèse est un patient humain. Le dispositif d'arthrodèse de cheville selon l'invention est, plus spécifiquement et avantageusement, un dispositif d'arthrodèse tibiotalocalcanéenne (TTC) par enclouage, de préférence transplantaire, et de préférence encore rétrograde. A titre d'exemple, le dispositif d'arthrodèse selon l'invention pourra avantageusement être mis en oeuvre pour traiter un patient atteint d'une forme évoluée d'arthrose dégénérative, d'arthrose post-traumatique, d'arthrite inflammatoire, de nécrose du talus, d'ostéoarthropathie de Charcot, voire même en cas d'échec d'une ou plusieurs opérations chirurgicales, telle qu'une opération d'arthroplastie totale (remplacement de l'articulation de la cheville par une prothèse totale de cheville (PTC).

Le dispositif d'arthrodèse selon l'invention comprend un clou 1 d'arthrodèse destiné à être implanté, de préférence par voie plantaire, dans un tibia Ti, un talus Ta et un calcanéus Ca d'un patient, en général au cours d'une opération chirurgicale réalisée sous anesthésie générale ou locorégionale. Avantageusement, l'articulation de la cheville considérée aura fait l'objet, préalablement à la mise en place du clou 1 dans le corps du patient, d'une préparation adéquate, et par exemple de coupes osseuses et d'une extraction d'éléments cartilagineux entre le tibia Ti et le talus Ta (articulation tibio-talienne) d'une part et entre le talus Ta et le calcanéus Ca (articulation talo-calcanéenne) d'autre part, de sorte à supprimer tout ou partie des surfaces articulaires naturelles de l'articulation de la cheville à traiter, pour favoriser la fusion osseuse. Egalement, un logement aura pu avantageusement être préalablement ménagé dans lesdits tibia Ti, talus Ta et calcanéus Ca, à l'aide de tout moyen connu, pour recevoir ledit clou 1.

On notera ici que, dans certains cas de figures, l'articulation de la cheville à traiter à l'aide du dispositif d'arthrodèse selon l'invention peut être dépourvue de talus en tant que tel, notamment suite à une ablation chirurgicale de ce dernier, trop dégradé pour être conservé en l'état. A ce titre, le terme « talus » utilisé dans la présente description désigne avantageusement le talus Ta en tant que corps osseux naturel (talus physiologique), mais peut, le cas échéant, renvoyer à l'espace articulaire vide correspondant au talus lorsque ce dernier est absent, ou encore à un implant talien de substitution (ou tout élément équivalent visant à combler l'espace articulaire formé par l'absence de talus).

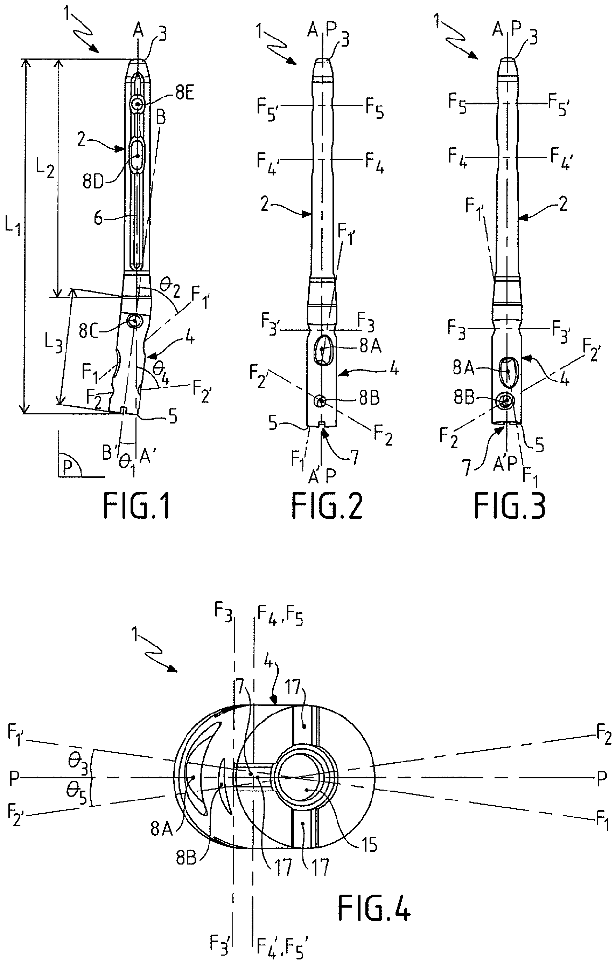

Un mode de réalisation préférentiel du clou 1 du dispositif selon l'invention est illustré aux figures 1 à 3. Le clou 1 illustré en exemple sur ces figures est destiné à être mis en place au niveau de l'articulation d'une cheville droite d'un patient. Bien évidemment, l'invention recouvre également un dispositif d'arthrodèse comprenant un clou qui serait destiné à être mis en place au niveau de l'articulation d'une cheville gauche d'un patient. Avantageusement, un tel clou pour cheville gauche serait défini par symétrie, par rapport au plan sagittal du patient, du clou 1 illustré aux figures.

Ledit clou 1 comprend, d'une part, une partie proximale 2, qui s'étend généralement selon une première direction d'extension A-A' longitudinale, de préférence à partir d'une extrémité proximale 3 libre du clou 1. D'autre part, le clou 1 comprend une partie distale 4, qui prolonge avantageusement ladite partie proximale 2, et s'étend généralement selon une deuxième direction d'extension B-B' longitudinale, de préférence jusqu'à une extrémité distale 5 libre du clou 1, opposée à ladite extrémité proximale 3.

Selon le mode de réalisation préférentiel illustré aux figures, lesdites partie proximale 2 et partie distale 4 sont toutes les deux allongées et préférentiellement sensiblement rectilignes, respectivement selon lesdites première A-A' et deuxième B-B' directions d'extension. Tel qu'illustré aux figures 1 à 3, le clou 1 se présente préférentiellement sous la forme générale d'une tige, de préférence pleine.

De préférence, la partie proximale 2 ainsi que, de préférence encore, la partie distale 3 du clou 1, présentent une forme générale cylindrique (à section circulaire). Alternativement, la partie proximale 2 et la partie distale 3 pourraient présenter une forme générale conique (à section circulaire), ou l'une des parties proximale 2 et partie distale 3 pourraient présenter une forme générale conique tandis que l'autre présente une forme générale cylindrique. De telle forme générale cylindrique et / ou conique facilitent avantageusement la mise en place du clou 1 à travers les corps osseux et tissus concernés.

De manière préférentielle, la partie proximale 2 du clou 1 est pourvue d'un moyen de blocage en rotation du clou 1 autour de ladite première direction d'extension A-A'. De préférence, ledit moyen de blocage comprend au moins une rainure 6 ou ailette longitudinale. Cependant, ledit moyen de blocage pourra alternativement comprendre au moins méplat longitudinal ou tout autre moyen adapté. Dans l'exemple illustré aux figures, la partie proximale 2 du clou 1 comprend deux rainures 6 longitudinales, positionnées de manière symétrique relativement à la première direction d'extension A-A' de ladite partie proximale 2. Avantageusement, de telles rainures longitudinales 6 peuvent en outre permettre de réduire les efforts exercés par le clou 1 dans le tibia Ti, en réduisant localement le volume d'encombrement du clou 1.

Bien évidemment, le clou 1 pourra éventuellement présenter une forme générale différente de celle exposée ci-dessus, par exemple celle d'une tige de section ovale ou polyédrique. Egalement, lesdites parties proximale 2 et distale 4 pourraient présenter des sections de profils différents l'une de l'autre.

De préférence, le clou 1 est monolithique et forme donc une pièce monobloc, d'un seul tenant. Il peut s'agir d'une pièce moulée et / ou usinée. Avantageusement, ledit clou 1 est réalisé en un matériau biocompatible (par nature ou rendu biocompatible à la suite d'un traitement) et apte à supporter les contraintes biomécaniques caractéristiques d'une cheville, tel que par exemple en acier inox, en alliage de titane, en alliage chrome-cobalt, ou encore en un matériau polymère, tel que par exemple le polyéther-éther-cétone (PEEK), chargé ou non.

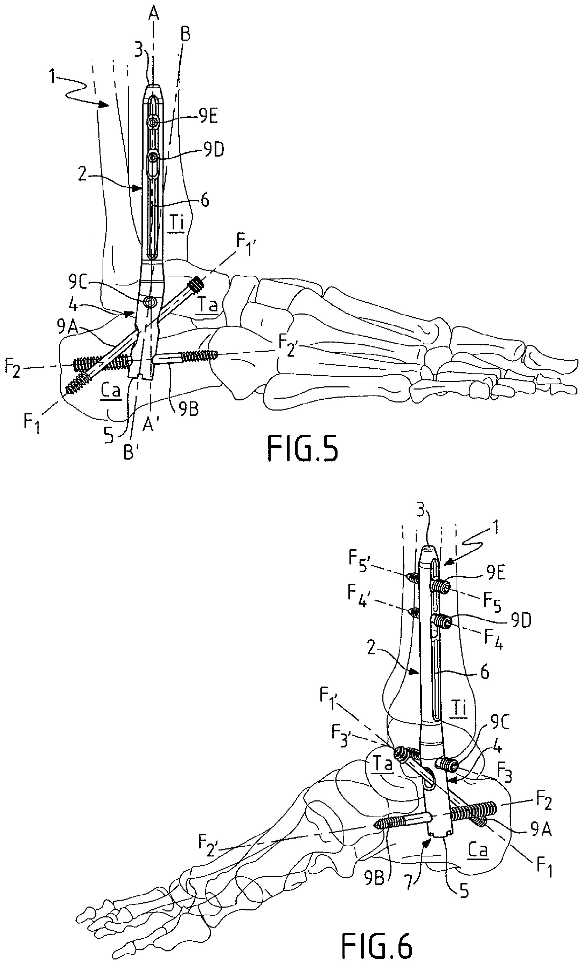

De préférence, la partie proximale 2 du clou 1 est prévue pour être majoritairement insérée et positionnée dans le tibia Ti du patient, de préférence dans le canal médullaire de ce dernier (clou centromédullaire), de telle sorte que la première direction A-A' d'extension de ladite partie proximale 2 du clou 1 est sensiblement parallèle à (si non confondue avec) l'axe d'extension verticale du tibia Ti. La partie distale 4 du clou 1 est quant à elle préférentiellement prévue pour être majoritairement insérée et positionnée dans le calcanéus Ca correspondant du patient (figure 5). Tel qu'illustré aux figures, lesdites parties proximale 2 et distale 4 peuvent avantageusement toutes deux être insérées et positionnées en partie, de façon minoritaire, dans le talus Ta.

Avantageusement, l'extrémité proximale 3 du clou 1 pourra être arrondie ou chanfreinée afin de faciliter l'insertion et le guidage du clou 1 au sein des calcanéus Ca, talus Ta et tibia Ti concernés.

Selon l'invention, les première A-A' et deuxième B-B' directions respectives des parties proximale 2 et distale 4 du clou 1 sont sécantes, c'est-à-dire que le clou 1 est courbé, tel que cela peut être observé aux figures 1 à 3 notamment. Les extrémités proximale 3 et distale 5 libres du clou 1 présentent ainsi avantageusement entre elles un décalage. Avantageusement, lorsque le clou 1 du dispositif d'arthrodèse selon l'invention est implanté de manière à ce que la première direction A-A' d'extension de la partie proximale 2 du clou 1 est sensiblement parallèle à (si non confondue avec) l'axe d'extension verticale du tibia Ti, la partie distale 4 du clou 1 peut alors s'étendre à travers le calcanéus Ca de manière oblique par rapport à l'axe d'extension verticale du tibia Ti (figure 5). Une telle angulation permet d'augmenter avantageusement la longueur de parcours de la partie distale 4 du clou 1 au sein du calcanéus Ca, ce qui confère audit clou 1 une stabilité améliorée, au moins au niveau calcanéus Ca concerné.

De manière avantageuse, les première A-A' et deuxième B-B' directions d'extension respectives des parties proximale 2 et distale 4 sont sécantes selon un premier angle θ₁ compris entre 5 et 10° ± 2°, et de préférence égal à 7° ± 2°. Une telle valeur angulaire apporte en effet avantageusement un très bon compromis en matière de stabilité mécanique conférée au clou 1 par l'angulation, sans impacter outre mesure la facilité de mise en place dudit clou 1 au sein de l'articulation de la cheville de patient.

Dans le mode de réalisation préférentiel illustré aux figures, le clou 1 est conçu et dimensionné de sorte que, lorsque ledit clou 1 est implanté dans l'articulation de la cheville, le point d'intersection des première A-A' et deuxième B-B' directions d'extension respectives des parties proximale 2 et distale 4 du clou 1, situé au voisinage au niveau de la jonction de ces dernières, est positionné au sein du talus Ta correspondant du patient (figures 5 à 8).

Afin de permettre la fixation et le maintien du clou 1 en position dans l'articulation de la cheville, une fois ledit clou 1 inséré dans lesdits tibia Ti, talus Ta et calcanéus Ca, la partie distale 4 du clou 1 est pourvue d'au moins un premier orifice de fixation 8A. Ce dernier traverse le clou 1 de part en part, et est destiné à recevoir et à guider un premier moyen de fixation 9A du clou 1 dans l'articulation de la cheville. Selon l'invention, ledit premier moyen de fixation 9A est du type vis, tel qu'on le verra dans ce qui suit.

Selon l'invention, le clou 1 est plus spécifiquement et avantageusement conçu et configuré de sorte que, lorsque ledit clou 1 est implanté avec ladite partie distale 4 orientée vers l'arrière de la cheville du patient (figures 5 et 6), ledit premier orifice de fixation 8A est agencé, et en particulier orienté, pour recevoir et guider ledit premier moyen de fixation 9A selon une première direction de fixation F₁-F₁' qui s'étend à travers lesdits talus Ta et calcanéus Ca, pour fixer ledit clou 1 aux talus Ta et calcanéus Ca en question. De préférence, l'axe principal d'extension du premier orifice de fixation 8A à travers le clou 1 est confondu avec ladite première direction de fixation F₁-F₁'. Comme cela est visible aux figures, ledit premier orifice de fixation 8A débouche d'une face avant et d'une face arrière opposée de la partie distale 4 du clou 1 (en considération de l'avant et de l'arrière de la cheville lorsque le clou 1 est implanté), et s'étend avantageusement en oblique relativement à la deuxième direction B-B' de la partie distale 4 du clou 1. Il forme ainsi à travers le clou 1 un conduit dont l'axe principal d'extension à travers le clou 1 est oblique par rapport à ladite deuxième direction B-B'.

Grâce à cette configuration particulière du clou 1, le premier moyen de fixation 9A, dimensionné de manière convenable, est avantageusement apte à constituer un moyen de fixation transarticulaire du clou 1, qui permet de fixer le clou 1 dans l'articulation de la cheville, par coopération desdits premier orifice de fixation 8A et premier moyen de fixation 9A, en immobilisant l'articulation talo-calcanéenne (ou articulation sous-talienne).

L'invention réside ainsi essentiellement dans l'idée d'un dispositif d'arthrodèse de cheville qui comprend un clou 1 présentant à la fois une forme coudée qui permet à sa partie distale de s'étendre dans le calcanéus Ca vers l'arrière de la cheville pour assurer la stabilité du clou 1 dans le calcanéus Ca, et un premier orifice de fixation 8A conçu pour recevoir et guider un premier moyen de fixation 9A transarticulaire de manière à ce que ce dernier puisse traverser le clou 1 de part en part pour assurer la solidarisation du calcanéeus Ca au talus Ta (immobilisation de l'articulation sous-talienne). Cette combinaison astucieuse et inédite de caractéristiques confère au clou 1 du dispositif d'arthrodèse selon l'invention une excellente stabilité mécanique en usage et permet un maintien des corps osseux de l'articulation de la cheville qui est particulièrement favorable à la réalisation d'une bonne fusion osseuse.

Selon l'invention, ledit premier orifice de fixation 8A est oblong selon ladite deuxième direction d'extension B-B' de la partie distale 4 du clou 1. Ainsi, la partie distale 4 du clou 1 conserve un certain degré de mobilité en translation relativement au premier moyen de fixation 9A, selon ladite deuxième direction d'extension B-B', lorsque le moyen de fixation 9A est reçu et guidé dans le premier orifice de fixation 8A. Avantageusement, le grand diamètre (mesuré selon la deuxième direction d'extension B-B') dudit premier orifice de fixation 8A est compris entre 5 et 15 mm ± 1 mm, de préférence égal à 13 mm ± 1 mm.

Selon l'invention, le dispositif d'arthrodèse comprend, outre le clou 1, ledit premier moyen de fixation 9A, lequel est de type vis, telle que classiquement connue et utilisée dans le domaine.

Selon une variante (non illustrée) non conforme à l'invention telle que revendiquée, ledit premier moyen de fixation 9A est du type vis non compressive.

Selon l'invention, ledit premier moyen de fixation 9A est, au contraire, du type vis compressive 9A d'ostéosynthèse. En d'autres termes, ledit premier moyen de fixation 9A est, selon l'invention, un moyen de fixation compressif, c'est-à-dire un moyen conçu pour permettre la fixation relative de deux corps osseux tout en assurant un effet de rapprochement et de mise en compression de ces corps osseux lors de la mise en place du moyen de fixation. Un tel premier moyen de fixation 9A est, selon l'invention, une vis compressive 9A d'ostéosynthèse, comme illustré aux figures. Avantageusement, une telle vis compressive possède au niveau de sa tête un premier filetage avec un premier pas *p₁* constant et, au niveau de sa partie distale, un deuxième filetage avec un deuxième pas *p₂* constant, le premier pas *p₁* étant inférieur au deuxième pas *p₂*. Comme illustré, la vis compressive peut comprendre une portion non filetée, qui s'étend entre les premier et deuxième filetages. Aussi parfois dénommée « *vis de compression à os* », une telle vis compressive 9A est connue en tant que telle, et peut éventuellement être de conception différente de celle illustrée aux figures. Dans certaines variantes non revendiquées, un tel moyen de fixation compressif n'est pas nécessairement une vis, et peut être de tout autre type adéquat.

La mise en oeuvre d'un tel premier moyen de fixation 9A compressif en combinaison du clou 1 présenté ci-avant permet une meilleure compression de l'articulation sous-talienne. Avantageusement, ladite vis compressive 9A pourra être canulée, pour en faciliter la mise en place, à l'aide par exemple d'une broche de Kirschner. Selon l'invention le premier orifice de fixation 8A correspondant est oblong et ladite vis compressive 9A pourra avantageusement, si besoin, se déplacer dans le premier orifice de fixation 8A du clou 1 lors d'une opération ultérieure de mise en compression des corps osseux concernés. Ainsi, la compression de l'articulation de la cheville pourra avantageusement réalisée sans solliciter le clou 1 en lui-même.

Alternativement encore, mais de manière toutefois moins avantageuse et non conforme à l'invention telle que revendiquée, ledit premier moyen de fixation 9A pourra éventuellement être d'un autre type convenable, par exemple de type broche ou clou.

Afin de s'adapter au mieux à l'anatomie (et en particulier à la longueur du tibia Ti) du patient, le clou 1 présente de préférence une longueur totale L₁, mesurée entre ses extrémités proximale 3 et distale 5, comprise entre 150 et 350 mm. De préférence, la partie proximale 2 du clou 1 présente une longueur L₂, mesurée selon ladite première direction d'extension A-A', sensiblement comprise entre 100 et 300 mm, tandis que la partie distale 4 du clou 1 présente une longueur L₃, mesurée selon ladite deuxième direction d'extension B-B', sensiblement comprise entre 45 et 55 mm. De préférence, les parties proximale 2 et distale 4 présentent un diamètre compris entre 10 mm et 13 mm ± 1 mm. De préférence encore, la partie distale 4 présente un diamètre supérieur au diamètre respectif de la partie proximale 2.

Comme cela va être présenté ci-après, le clou 1 peut avantageusement comprendre un ou plusieurs autres orifices de fixation, chacun destiné à recevoir et à guider un moyen de fixation supplémentaire, distinct dudit premier moyen de fixation 9A présenté ci-avant, afin de contribuer au bon ancrage du clou 1 dans les corps osseux de l'articulation de la cheville concernée.

De préférence, ladite partie distale 4 du clou 1 est pourvue d'un deuxième orifice de fixation 8B (ou orifice de fixation calcanéenne), qui est configuré pour recevoir et guider un deuxième moyen de fixation 9B selon une deuxième direction de fixation F₂-F₂' pour fixer ledit clou 1 au calcanéus Ca concerné du patient. Avantageusement, ledit deuxième orifice de fixation 8B est configuré de sorte à recevoir et guider ledit deuxième moyen de fixation 9B pour fixer le clou 1 au seul dit calcanéus Ca, c'est-à-dire de sorte que ledit deuxième moyen de fixation 9B ne puisse coopérer avec le talus Ta correspondant. Dans l'exemple illustré aux figures, ledit deuxième orifice de fixation 8B est positionné à proximité de l'extrémité distale 5 libre du clou 1, entre ledit premier orifice de fixation 8A et l'extrémité distale 5 libre du clou 1.

En autorisant avantageusement une implantation du clou 1 avec la partie distale 4 de ce dernier orientée vers l'arrière de la cheville du patient, comme évoqué ci-avant, la courbure de la partie distale 4 du clou 1 par rapport à la partie proximale 2 de ce dernier permet avantageusement un meilleur centrage longitudinal du deuxième orifice de fixation 8B au sein du calcanéus Ca. De la sorte, ledit deuxième orifice de fixation 8B est rapproché de l'extrémité postérieure du calcanéus, par rapport notamment au cas d'un clou qui serait strictement rectiligne, ce qui facilite la mise en place dudit deuxième moyen de fixation 8B par l'arrière du calcanéus (voie d'abord globalement postérieure). D'autre part, une telle configuration permet de ménager avantageusement une plus grande zone osseuse disponible pour le parcours du deuxième moyen de fixation 8B dans la portion antérieure du calcanéus Ca. L'ancrage de la partie distale 4 du clou 1 au calcanéus Ca s'en trouve ainsi amélioré.

De préférence, ledit deuxième orifice de fixation 8B s'étend en oblique relativement à la deuxième direction B-B' de la partie distale 4 du clou 1, c'est-à-dire qu'il forme un conduit dont l'axe principal d'extension est oblique par rapport à ladite deuxième direction B-B'. De préférence, ledit deuxième orifice de fixation 8B est sensiblement cylindrique et l'axe principal d'extension de ce dernier à travers le clou 1 est confondu avec ladite deuxième direction de fixation F₂-F₂'.

De préférence, ladite partie distale 4 du clou 1 est pourvue d'un troisième orifice de fixation 8C (ou orifice de fixation talaire), qui est configuré pour recevoir et guider un troisième moyen de fixation 9C selon une troisième direction de fixation F₃-F₃' pour fixer ledit clou 1 au talus Ta concerné du patient. De préférence, l'axe d'extension principal dudit troisième orifice de fixation 8C à travers le clou 1 est confondu avec ladite troisième direction de fixation F₃-F₃'. De préférence, ladite troisième direction de fixation F₃-F₃' est orthogonale à la deuxième direction d'extension B-B' de la partie distale 4 du clou 1. Avantageusement, ledit troisième orifice de fixation 8C est configuré de sorte à recevoir et guider ledit troisième moyen de fixation 9C pour fixer le clou 1 au seul dit talus Ta, c'est-à-dire de sorte que ledit troisième moyen de fixation 9C ne puisse coopérer avec le calcanéus Ca correspondant.

Dans le mode de réalisation préférentiel illustré aux figures, ledit troisième orifice de fixation 9C est agencé au voisinage de la zone de jonction entre lesdites parties proximale 2 et distale 4 du clou 1 et du point d'intersection des premières A-A' et deuxième B-B' directions d'extension, ledit premier orifice fixation 8A étant agencé entre ledit troisième orifice de fixation 8C et l'extrémité distale 5 libre du clou 1.

De préférence, la partie proximale 2 du clou 1 est pourvue d'un quatrième orifice de fixation 8D (premier orifice de fixation tibiale), qui est configuré pour recevoir et guider un quatrième moyen de fixation 9D selon une quatrième direction de fixation F₄-F₄' pour fixer ledit clou 1 au tibia Ti concerné du patient. De préférence, ladite quatrième direction de fixation F₄-F₄' est orthogonale à la première direction d'extension A-A' de la partie proximale 2 du clou 1. Avantageusement, l'axe d'extension principal dudit quatrième orifice de fixation 8D à travers le clou 1 est confondu avec ladite quatrième direction de fixation F₄-F₄'.

De préférence encore, la partie proximale 4 est pourvue d'un cinquième orifice de fixation 8E (deuxième orifice de fixation tibiale), distinct dudit quatrième orifice de fixation 8D, et qui est configuré pour recevoir et guider un cinquième moyen de fixation 9E selon une cinquième direction de fixation F₅-F₅', pour fixer lui aussi ledit clou 1 audit tibia Ti. De préférence, ladite cinquième direction de fixation F₅-F₅' est orthogonale à la première direction d'extension A-A' de la partie proximale 2 du clou 1. Avantageusement, l'axe d'extension principal dudit cinquième orifice de fixation 8E à travers le clou 1 est confondu avec ladite cinquième direction de fixation F₅-F₅'. Une telle configuration à deux orifices de fixation tibiale 8D, 8E est préférée car elle garantit *a priori* un meilleur maintien de la partie proximale 2 du clou 1 au sein du tibia Ti.

Tel qu'illustré, lesdits quatrième 8D et cinquième 8E orifices de fixation sont positionnés l'un en dessous de l'autre selon la première direction d'extension A-A' de la partie proximale 2 du clou 1, de manière centrée par rapport à ladite première direction d'extension A-A'. De préférence, lesdits quatrième 8D et cinquième 8E orifices de fixation sont positionnés au niveau de la moitié la plus proximale de la partie proximale 2 du clou 1 de sorte à améliorer encore l'ancrage du clou dans le tibia Ti.

De préférence, l'un desdits quatrième 8D et cinquième 8E orifices de fixation est de forme oblongue selon ladite première direction d'extension A-A'. Le cas échéant, l'autre des quatrième 8D et cinquième 8E orifices de fixation pourra quant à lui être par exemple circulaire. Dans l'exemple illustré aux figures, l'orifice de fixation de forme oblongue est celui qui, parmi les quatrième 8D et cinquième 8E orifices de fixation, est le plus éloigné de l'extrémité proximale 3 du clou 1.

Un tel orifice de fixation de forme oblongue permet avantageusement, en combinaison avec le moyen de fixation correspondant, de fixer la partie proximale 2 du clou 1 au tibia Ti tout en conservant une certaine latitude de translation dudit clou 1 au sein dudit tibia Ti (fixation dynamique), en particulier en vue d'une opération de compression de l'articulation de la cheville, conduite par le chirurgien, et visant à rapprocher le calcanéus Ca du tibia Ti. La combinaison préférentielle, illustrée aux figures, d'un quatrième 8D orifice de fixation oblong avec un cinquième orifice de fixation 8E non oblong permet avantageusement de fixer, dans un premier temps, la partie proximale 2 du clou 1 au tibia Ti de manière dynamique (par coopération desdits quatrième orifice de fixation 8D et quatrième moyen de fixation 9D), en vue notamment d'une opération ultérieure de compression de l'articulation, avant de verrouiller complètement ladite partie proximale 2 au tibia Ti, par coopération desdits cinquième orifice de fixation 8E et cinquième moyen de fixation 9E de fixation. Avantageusement, le grand diamètre (mesuré selon la première direction d'extension A-A') de ce(s) quatrième 8D et / ou cinquième 8E orifice(s) de fixation oblong(s) est compris entre 5 et 15 mm ± 1 mm, de préférence égal à 13 mm ± 1 mm environ.

De manière avantageuse, le dispositif d'arthrodèse selon l'invention comprend tous ou partie des deuxième 9B, troisième 9C, quatrième 9D et cinquième 9E moyens de fixation évoqués ci-dessus. Dans l'exemple illustré notamment aux figures 5 et 6, ces moyens de fixation 9B, 9C, 9D, 9E sont de type deuxième 9B, troisième 9C, quatrième 9D et cinquième 9E vis de fixation, telles que classiquement connues et utilisées dans le domaine. Eventuellement, lesdites vis de fixation 9B, 9C, 9D, 9E pourront être canulées pour en faciliter la mise en place, à l'aide par exemple d'une broche de Kirschner. Ceci étant, tous ou partie desdits deuxième 9B, troisième 9C, quatrième 9D et cinquième 9E moyens de fixation pourraient alternativement être du type clou, agrafe, broche ou autre.

De préférence, lesdites premières A-A' et deuxième B-B' directions d'extension sont inscrites dans un même plan principal P, tel qu'illustré en particulier à la figure 1. Les parties proximale 2 et distale 4 du clou 1 sont alors avantageusement coplanaires. De préférence encore, ledit clou 1 est configuré et dimensionné de sorte que, lorsque ledit clou 1 est implanté dans l'articulation de la cheville du patient, ledit plan principal P est sensiblement parallèle au plan sagittal du patient. En d'autres termes, le clou 1 est avantageusement conformé, configuré, dimensionné, de manière à ce que les première A-A' et deuxième B-B' directions d'extension respectives des parties proximale 2 et distale 4 du clou 1 puisse être orientées parallèlement au plan sagittal du patient lors de l'implantation du clou 1, sans pour autant remettre en cause la fonction des orifices de fixation 8A, 8B, 8C, 8D, 8E évoqués ci-avant.

Ainsi, ledit clou 1 est avantageusement conçu pour pouvoir être implanté avec ladite partie distale 4 orientée vers l'arrière de la cheville selon une direction sensiblement antéro-postérieure. Lesdites extrémités proximale 3 et distale 5 présentent alors entre elles un décalage dans le plan principal P, l'extrémité distale 5 étant décalée en postérieur uniquement (et non en latéral, en médial ou encore en médio- / latéro-postérieur). En effet, il a été observé qu'une telle orientation est particulièrement bénéfique pour la stabilité du clou 1 au sein de l'articulation de la cheville traitée, ce qui favorise *in fine* une bonne ostéosynthèse des corps osseux de ladite articulation tout en contribuant au confort du patient.

Dans le mode de réalisation préférentiel illustré aux figures, les rainures 6 longitudinales qui, comme évoqué ci-avant, forment avantageusement un moyen de blocage en rotation du clou 1 autour de ladite première direction d'extension A-A', sont préférentiellement agencées de manière symétrique de part et d'autre dudit plan principal P, pour faciliter l'identification par le chirurgien de l'orientation du plan P dans l'espace.

Avantageusement, le clou 1 est pourvu d'un élément de repère 7 de l'orientation du plan principal P dans l'espace, afin d'aider le chirurgien à positionner ce dernier de manière sensiblement parallèle au plan sagittal du patient au cours de l'opération d'implantation du clou 1 dans l'articulation de la cheville. Dans l'exemple illustré aux figures, cet élément de repère 7 consiste préférentiellement en une encoche 7, qui est ménagée dans le clou 1 au niveau de son extrémité distale 5 libre et qui s'étend selon une direction principale parallèle au plan principal P (et de préférence, inscrite dans ledit plan principal P). De préférence, ladite encoche 7 débouche d'une seule des faces avant (i.e. destinée à être orientée vers l'avant de la cheville) et arrière (*i.e.* destinée à être orientée vers l'arrière de la cheville) du clou 1, par exemple de la face avant (figure 5), de sorte à renseigner le chirurgien non seulement sur l'orientation du plan principal P dans l'espace, mais également sur l'orientation de la partie distale 4 du clou 1. Bien évidemment, ladite encoche 7 pourra être remplacée tout autre moyen d'élément de repère convenable.

De manière plus préférentielle encore, ledit clou 1 est configuré de sorte que, lorsque ledit clou 1 est implanté dans l'articulation de la cheville du patient, ledit plan principal P est alors confondu avec un plan sensiblement vertical comprenant l'axe principal moyen du col du calcanéus Ca concerné. Une telle configuration permet avantageusement d'obtenir un bon centrage médio-latéral de la partie distale 4 du clou 1 dans la masse osseuse du calcanéus Ca.

De préférence, ladite première direction de fixation F₁-F₁' est :
- d'une part, sécante à ladite première direction d'extension A-A' de la partie proximale 2 du clou 1 selon un deuxième angle θ₂ de préférence égal à 45° ± 3° (figure 1), et
- d'autre part, sécante audit plan principal P selon un troisième angle θ₃ de préférence égal à 9° ± 2° (figure 4).

Ainsi, une fois le clou 1 implanté, ledit premier moyen de fixation 9A peut être mis en place dans le talus Ta et le calcanéus Ca du patient par l'intermédiaire du premier orifice de fixation 8A, selon ladite première direction F₁-F₁' qui est alors orientée de manière descendante oblique de l'avant et de l'intérieur de la cheville (antéro-médial) vers l'arrière et l'extérieur de cette dernière (postéro-latéral). Compte tenu des morphologies respectives du calcanéus Ca et du talus Ta, une telle orientation de la première direction de fixation F₁-F₁' permet d'optimiser avantageusement la longueur du parcours disponible pour le premier moyen de fixation 9A au sein de la masse osseuse respective desdits calcanéus Ca et talus Ta, et donc d'obtenir un ancrage optimal du clou 1 dans ces derniers par l'intermédiaire dudit premier moyen de fixation 9A. Entre, une telle configuration du premier orifice de fixation 8A autorise avantageusement la mise en place dudit premier moyen de fixation 9A par l'avant de la cheville, en particulier par la face avant supérieure du talus Ta (figures 5 et 6).

De préférence, ladite deuxième direction de fixation F₂-F₂' est :
- d'une part, sécante à ladite première direction A-A' de la partie proximale 2 du clou 1 selon un quatrième angle θ₄ égal à 78° ± 3°, et
- d'autre part, sécante audit plan principal P selon un cinquième angle θ₅ égal à 8° ± 2°.

Ainsi, lorsque le clou 1 est implanté, ledit deuxième moyen de fixation 8B peut être mis en place au sein du calcanéus Ca du patient, selon ladite deuxième direction F₂-F₂' qui est alors orientée de manière ascendante oblique de l'arrière et de l'extérieur de la cheville (postéro-latéral) vers l'avant et l'intérieur (antéro-médial) de cette dernière. Ladite deuxième direction de fixation F₂-F₂' est alors préférentiellement inscrite dans un plan vertical qui est sécant à le plan vertical correspondant dans lequel est inscrit la première direction de fixation F₁-F₁'. Compte tenu de la morphologie générale du calcanéus Ca, une telle orientation de la deuxième direction de fixation F₂-F₂' permet avantageusement d'optimiser la longueur du parcours disponible pour le deuxième moyen de fixation 8B au sein de la masse osseuse dudit calcanéus Ca, et donc d'obtenir un ancrage optimal du clou 1 dans ce dernier par l'intermédiaire dudit deuxième moyen de fixation 9B.

De préférence, ladite troisième direction de fixation F₃-F₃' est orthogonale audit plan principal P, dans lequel sont préférentiellement inscrites les premières A-A' et deuxième B-B' directions d'extension respectives des parties proximale 2 et distale 4 du clou 1. Ainsi, lorsque le clou 1 est implanté, ledit plan principal P étant sensiblement parallèle au plan sagittal du patient, la troisième direction de fixation F₃-F₃' constitue avantageusement une direction de fixation talaire médio-latérale. La coopération de ce troisième orifice de fixation 8C du clou 1 avec ledit troisième moyen de fixation 9C contribue avantageusement à immobiliser l'articulation tibio-talaire de la cheville.

De préférence, ladite quatrième direction de fixation F₄-F₄' est orthogonale audit plan principal P, dans lequel sont préférentiellement inscrites les premières A-A' et deuxième B-B' directions d'extension respectives des parties proximale 2 et distale 4 du clou 1. De préférence, ladite cinquième direction de fixation F₅-F₅' est parallèle à ladite quatrième direction de fixation F₄-F₄', ce qui permet de simplifier la mise en place et la fixation du clou 1 par le chirurgien.

Dans le mode de réalisation préférentiel illustré aux figures, les quatrième 8D et cinquième 8E orifices de fixation sont orientés de sorte que lesdites quatrième F₄-F₄' et cinquième F₅-F₅' directions de fixation correspondantes sont parallèles entre elles et orthogonales audit plan principal P. Ainsi, lorsque le clou 1 est implanté, ledit plan principal P étant sensiblement parallèle au plan sagittal du patient, les quatrième F₄-F₄' et cinquième F₅-F₅' directions de fixation constituent avantageusement respectivement des première et deuxième directions de fixation tibiale médio-latérale.

Le dispositif d'ostéosynthèse selon l'invention pourra éventuellement comprendre une pluralité de clous conformes à la description qui en a été faite ci-avant, et constituer ainsi avantageusement un kit d'ostéosynthèse. Ces clous pourront présenter une partie distale respective identique et une partie proximale respective de longueur L₂ et / ou de diamètre différents. En outre, lesdits clous pourront être proposés dans une version droite (telle qu'illustrée aux figures) et dans une version gauche. Un tel kit permettra au chirurgien de s'adapter au mieux à l'anatomie du patient à traiter, et en particulier à l'anatomie du calcanéus, du talus et du tibia de ce dernier.

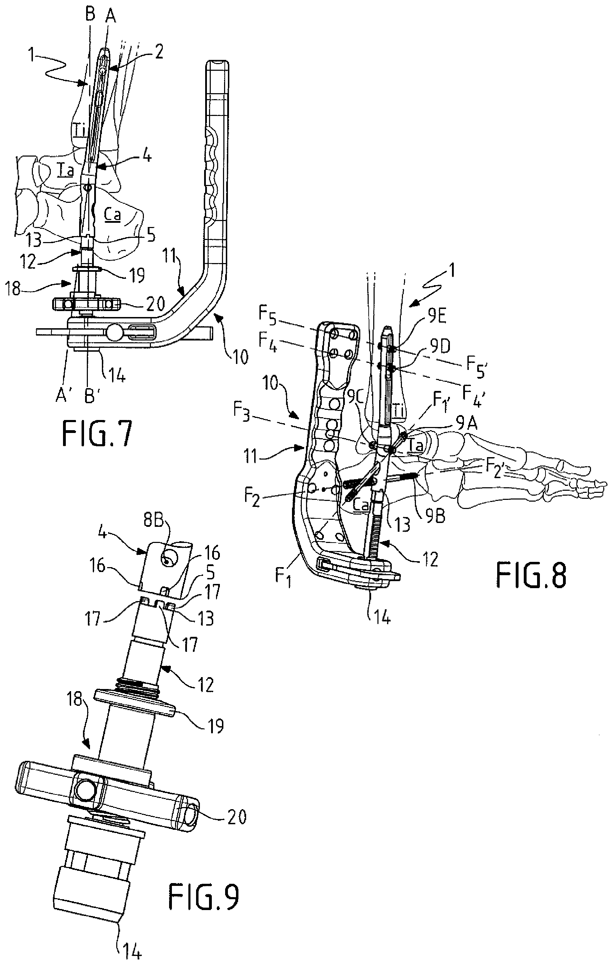

De manière préférentielle, le dispositif d'ostéosynthèse selon l'invention comprend en outre un instrument 10 de mise en place dudit clou 1 au sein de ladite articulation de la cheville du patient, de préférence par voie plantaire. Un tel instrument 10 est représenté aux figures 7 et 8, en combinaison avec le clou 1. De préférence, l'instrument 10 comprend un bras 11, avantageusement préhensible manuellement par le chirurgien. De préférence, ledit instrument 10 est conçu pour être accouplé de manière amovible à l'extrémité distale 5 dudit clou 1. A ce titre, l'instrument 10 comprend avantageusement un moyen d'interfaçage 12 (ou connecteur) amovible pour relier le bras 10 au clou 1. De préférence, ledit moyen d'interfaçage 12 est lui-même conçu pour être assemblé de manière amovible au bras 11, de sorte qu'il est possible d'assembler dans un premier temps le moyen d'interfaçage 12 au clou 1, puis d'assembler dans un deuxième temps le moyen d'interfaçage 12 au bras 11.

Tel qu'illustré en particulier à la figure 8, ledit moyen d'interfaçage 12 forme une pièce sensiblement tubulaire, qui s'étend de préférence longitudinalement entre une première 13 et une deuxième 14 extrémités. Ledit moyen d'interfaçage 12 comprend de préférence une vis de connexion (non illustrée) interne montée axialement au sein du moyen d'interfaçage 12, et dont une partie distale filetée se projette hors du moyen d'interfaçage 12 à partir de la première extrémité 13 de ce dernier, de sorte à former au niveau de ladite première extrémité 13 un embout fileté. Avantageusement, la tête de la vis de connexion est accessible par l'intermédiaire de ladite deuxième extrémité 14.

De préférence, le clou 1 est réciproquement pourvu d'un trou taraudé 15 prévu pour coopérer avec ladite vis de connexion du moyen d'interfaçage 12 pour accoupler ce dernier, et donc l'instrument 10, à l'extrémité distale 5 libre dudit clou 1. Ledit trou taraudé 15, visible en particulier à la figure 4, s'étend avantageusement à travers la partie distale 4 du clou 1, depuis l'extrémité distale 5 libre de ce dernier, et de manière coaxiale avec la deuxième direction d'extension B-B' de ladite partie distale 4. Tel qu'illustré aux figures 7 et 8, le clou 1 repose ainsi avantageusement sur le moyen d'interfaçage 12, la deuxième direction d'extension B-B' de la partie distale 4 du clou 1 étant alignée avec l'axe d'extension longitudinale correspondant du moyen d'interfaçage 12, de telle sorte qu'un déplacement du moyen d'interfaçage 12 entraîne un déplacement sensiblement identique du clou 1.

Afin de faciliter le positionnement et l'accouplement du clou 1 relativement au moyen d'interfaçage 12 et de limiter le risque pour le chirurgien de se tromper dans l'orientation du clou 1, le moyen d'interfaçage 12 et le clou 1 comportent avantageusement des moyens de repérage 16, 17 complémentaires. Tel que cela est représenté aux figures 4 et 9, les moyens de repérage 16, 17 sont de préférence formés par trois ergots 16 et trois encoches 17 agencés de telle manière que chaque encoche 17 ne puisse coopérer qu'avec un seul ergot 16 correspondant. De préférence, les encoches 17 sont ménagées dans l'extrémité distale 5 libre du clou 1 destinée à venir en contact avec la première extrémité 13 correspondante du moyen d'interfaçage 12 dans laquelle sont quant à eux ménagés les ergots 16. Bien évidemment, une configuration inversée, dans laquelle le clou 1 serait muni d'ergots destinés à coopérer avec des encoches correspondantes ménagées sur le moyen de d'interfaçage 12, est tout à fait envisageable. De préférence, les moyens de repérage 16, 17 comprennent donc trois paires formées chacune par un ergot 16 et une encoche 17 complémentaires, lesdites paires étant situées sur le pourtour de la jonction entre le clou 1 et le moyen d'interfaçage 12, et orientées sensiblement à 90 degrés les unes par rapport aux autres. Une telle configuration des moyens de repérage 16, 17 permet notamment d'éviter que le chirurgien n'assemble le clou 1 relativement à l'instrument 10 dans une autre orientation que celle prévue, et en particulier selon une orientation telle, qu'une fois le clou 1 implanté, la partie distale 4 de ce dernier ne serait pas dirigée vers l'arrière de la cheville du patient.

De préférence, l'une desdites encoches 17 est confondue avec l'encoche 7 évoquée précédemment (figure 4), qui forme avantageusement un élément de repère 7 de l'orientation du plan principal P du clou 1. Avantageusement, les autres encoches 17 sont alors alignées selon une direction orthogonale au plan principal P (figure 4). Ainsi, lesdits moyens de repérage 16, 17 contribuent avantageusement à guider le chirurgien lors de l'implantation du clou 1 dans l'articulation de la cheville du patient, de sorte en particulier que ce dernier puisse positionner le clou 1 de manière à ce que ledit plan principal P de ce dernier soit parallèle au plan sagittal du patient.

Tel que cela est représenté sur les figures 7 et 8, le clou 1 peut ainsi avantageusement être relié au moyen d'interfaçage 12 par la première extrémité 13 de ce dernier, pour venir occuper une position stable et fixe d'insertion du clou 1 dans le corps du patient.

Tel qu'illustré aux figures 7 et 9, ledit instrument 10 comprend avantageusement un moyen de mise en compression 18 (omis sur la figure 8) de l'articulation de la cheville, destiné à permettre le rapprochement du calcanéus Ca et du tibia Ti formant ladite articulation. De préférence, ledit système de mise en compression 18 comprend une portée 19 conçue pour venir en appui contre le contre la plante du pied du patient, en regard du calcanéus Ca, et susceptible d'être déplacée pour exercer une pression à l'encontre de ce dernier, de sorte à provoquer ainsi le rapprochement du calcanéus Ca du tibia Ti, et de préférence le rapprochement du calcanéus Ca et du talus Ta du tibia Ti.

De préférence, ladite portée 19 est montée selon une liaison glissière relativement au moyen d'interfaçage 12 de sorte à évoluer, de préférence selon une direction colinéaire à l'axe d'extension longitudinale du moyen d'interfaçage 12, entre au moins une position inactive (figure 7), dans laquelle la portée 19 n'est pas en contact avec la plante du pied du patient, et au moins une position active (non illustrée), dans laquelle ladite portée vient en contact en appui contre la plante du pied du patient, en regard du calcanéus, et comprime l'articulation, rapprochant ainsi le calcanéus Ca du tibia Ti.

Ledit moyen de mise en compression 18 comprend en outre de préférence un moyen de commande 20, par exemple de type volant 20 ou molette, conçu et agencé de manière à générer, par l'intermédiaire dudit moyen d'interfaçage 12, un déplacement de la portée 19 entre lesdites positions inactive et active. Avantageusement, ledit moyen de commande 20 est monté relativement au moyen d'interfaçage 12 selon une liaison hélicoïdale de direction confondue avec l'axe d'extension longitudinale du moyen d'interfaçage 12, de sorte que la mise en rotation du moyen de commande 20 autour de l'axe d'extension du moyen d'interfaçage 12 entraîne une translation de la portée selon ce même axe.

De manière préférentielle, ledit instrument 10 constitue un guide de perçage et / ou de visée pour la mise en place de tous ou partie des premier 9A, deuxième 9B, troisième 9C, quatrième 9D et cinquième 9E moyens de fixation du clou 1. Tel que cela est visible à la figure 9, le bras 11 de l'instrument 10 peut à ce titre être pourvu d'un certain nombre d'orifices de perçage et / ou de visée, qui sont configurés de sorte à former chacun à travers ledit bras 11 un conduit dont l'axe d'extension principal est colinéaire avec l'un au moins des première F₁-F₁', deuxième F₂-F₂', troisième F₃-F₃', quatrième F₄-F₄' et cinquième F₅-F₅' directions de fixations des premier 8A, deuxième 8B, troisième 8C, quatrième 8D et cinquième 8E, lorsque l'instrument 10 est accouplé au clou 1.

L'invention concerne également une méthode chirurgicale pour réaliser une arthrodèse de l'articulation de cheville, dans laquelle on utilise un dispositif d'arthrodèse, comprenant un clou 1 destiné à être implanté dans un tibia Ti, un talus Ta et un calcanéus Ca d'un patient, ledit clou 1 comprenant :
- une partie proximale 2 qui s'étend généralement selon une première direction d'extension A-A' longitudinale, et
- une partie distale 4 qui prolonge ladite partie proximale 2 et s'étend généralement selon une deuxième direction d'extension B-B' longitudinale, lesdites première A-A' et deuxième B-B' directions d'extension étant sécantes, ladite partie distale 4 étant pourvue d'un premier orifice de fixation 8A.

Avantageusement, ladite méthode chirurgicale met en oeuvre un dispositif d'arthrodèse conforme à celui selon l'invention, de sorte que la description qui été faite ci-avant de ce dernier reste applicable *mutatis mutandis* à la présente méthode chirurgicale. Ainsi, le clou 1 mis en oeuvre dans le cadre de ladite méthode chirurgicale est conçu et configuré de sorte que, lorsque ledit clou 1 est implanté avec ladite partie distale 4 orientée vers l'arrière de la cheville du patient, ledit premier orifice de fixation 8A est agencé, orienté, pour recevoir et guider un premier moyen de fixation 9A selon une première direction de fixation F1-F1' qui s'étend à travers lesdits talus Ta et calcanéus Ca pour fixer ledit clou 1 audit talus Ta et audit calcanéus Ca.

Ladite méthode chirurgicale comprend au moins une première étape au cours de laquelle on met en place ledit clou 1 dans l'articulation de la cheville du patient. Avantageusement, cette première étape de mise en place pourra être précédée d'une étape préparatoire au cours de laquelle on réalise, à l'aide de tout moyen connu (par exemple, à d'aide d'un foret canulé), un logement dans lesdits tibia Ti, talus Ta et calcanéus Ca, pour recevoir ultérieurement ledit clou 1, au cours de ladite première étape de mise en place de ce dernier. De préférence, ce logement est réalisé de manière à permettre la mise en mise en place dudit clou 1 par voie plantaire rétrograde. Cette étape préparatoire pourra éventuellement être précédée ou suivie d'une étape de préparation adéquate de l'articulation de la cheville à traiter, impliquant par exemple des coupes osseuses et une extraction d'éléments cartilagineux entre le tibia Ti et le talus Ta (articulation tibio-talienne) d'une part et entre le talus Ta et le calcanéus Ca (articulation talo-calcanéenne) d'autre part, de sorte à supprimer tout ou partie des surfaces articulaires naturelles de l'articulation de la cheville à traiter.

Au cours de ladite première étape de mise en place du clou 1, on implante ce dernier dans l'articulation de la cheville de manière à ce que ladite partie distale 4 du clou 1 soit orientée vers l'arrière de la cheville du patient.

De préférence, les première A-A' et deuxième B-B' directions d'extension respectives des parties proximale 2 et distale 4 du clou 1 mis en oeuvre par ladite méthode chirurgicale sont inscrites dans un même plan principal P. De manière préférentielle, on implante alors le clou 1, au cours de ladite première étape, de manière à ce que ladite partie distale 4 du clou 1 soit orientée vers l'arrière de la cheville du patient et à ce que ledit plan principal P soit sensiblement parallèle au plan sagittal du patient. De manière plus préférentielle encore, on implante alors le clou 1, au cours de ladite première étape, de manière à ce que ladite partie distale 4 du clou 1 soit orientée vers l'arrière de la cheville du patient et à ce que ledit plan principal P soit confondu avec un plan sensiblement vertical comprenant l'axe principal moyen du col du calcanéus Ca. La maîtrise d'une telle orientation peut être avantageusement facilitée par la mise en oeuvre de l'instrument 10 décrit ci-avant, en particulier par coopération des moyens de repérage 16, 17 complémentaires que comportent préférentiellement le moyen d'interfaçage 12 de l'instrument 10 et le clou 1, et / ou par contrôle radiographique.

De préférence, la première étape de mise en place dudit clou 1 est suivie d'une deuxième étape au cours de laquelle on fixe le clou 1 au tibia Ti, par l'intermédiaire d'un quatrième 8D et / ou d'un cinquième 8E orifice(s) de fixation, et à l'aide d'un quatrième 9D et / ou cinquième 9E moyens de fixation reçu(s) et guidé(s) dans le(s)dit(s) quatrième 8D et / ou cinquième 8E orifice(s) de fixation correspondant(s). De préférence, lesdits quatrième 9D et cinquième 9E moyens de fixation sont mis en place par voie médio-latérale.

De préférence, ladite deuxième étape de fixation du clou 1 au tibia Ti est suivie d'une troisième étape au cours de laquelle on met en compression l'articulation de la cheville, afin de rapprocher le calcanéus Ca du tibia Ti, et de préférence afin de rapprocher le calcanéus Ca et le talus Ta du tibia Ti. Cette troisième étape peut par exemple être réalisée manuellement, par pression sur le pied, ou à l'aide du moyen de mise en compression 20 de l'instrument 10 décrit précédemment en lien avec le dispositif d'arthrodèse selon l'invention.

De préférence, ladite troisième de mise en compression de l'articulation de la cheville est suivie d'une quatrième étape au cours de laquelle on fixe le clou 1 au talus Ta, par l'intermédiaire d'un troisième orifice de fixation 8C, et à l'aide d'un troisième moyen de fixation 9C reçu et guidé dans ledit troisième orifice de fixation 8C. De préférence, ledit troisième moyen de fixation 9C est mis en place par voie médio-latérale. Ladite quatrième étape vise ainsi avantageusement à fixer le clou 1 en immobilisant l'articulation tibio-talaire. De préférence, ledit troisième moyen de fixation 9C ne traverse alors (au moins partiellement) que le talus Ta.

De préférence, ladite quatrième étape de fixation du clou 1 au talus Ta est suivie d'une cinquième étape au cours de laquelle on fixe le clou 1 simultanément au talus Ta et au calcanéus Ca par l'intermédiaire dudit premier orifice de fixation 8A, et à l'aide dudit premier moyen de fixation 9A reçu et guidé dans ledit premier orifice de fixation 8A. Ladite cinquième étape vise ainsi avantageusement à fixer le clou 1 en immobilisant l'articulation talo-calcanéenne. Elle constitue avantageusement une étape de fixation transarticulaire du clou 1. Le premier moyen de fixation 9A utilisé au cours de ladite cinquième étape est de type vis compressive 9A. Ladite quatrième étape de fixation comprend alors avantageusement une opération de mise en compression de l'articulation talo-calcanéenne par serrage de ladite vis compressive 9A dans le talus Ta et le calcanéum Ca. De préférence, le premier moyen de fixation 9A est mis en place par l'avant de la cheville, de sorte qu'il traverse successivement (au moins partiellement) le talus Ta, le clou 1 et le calcanéus Ca.

De préférence, ladite cinquième étape de fixation simultanée du clou au talus Ta et au calcanéus Ca est suivie d'une sixième étape au cours de laquelle on fixe le clou 1 au calcanéus Ca par l'intermédiaire dudit deuxième orifice de fixation 8B, et à l'aide dudit deuxième moyen de fixation 9B reçu et guidé dans ledit deuxième orifice de fixation 8B. De préférence, ledit deuxième moyen de fixation 9B ne traverse alors (au moins partiellement) que le calcanéus Ca. De préférence, deuxième moyen de fixation 9B est mis en place par l'arrière de la cheville.

Bien évidemment, les différentes étapes décrites ci-dessus pourront être réalisées dans un ordre différent de celui qui vient d'être exposé. Certaines de ces étapes pourront par ailleurs être éventuellement omises ou modifiées. Par exemple, ladite première étape pourra être suivie de ladite cinquième étape, suivie de ladite quatrième étape, suivie de ladite deuxième étape, suivie enfin de ladite troisième étape.

Toutes ou partie des étapes décrites ci-dessus pourront avantageusement être en mises en oeuvre à l'aide de l'instrument 10 précédemment décrit en lien avec le dispositif d'arthrodèse selon l'invention.

On notera à toutes fins utiles que les termes « *premier* », « *deuxième* », « *troisième* », etc. utilisés dans la présente description n'ont de préférence aucune connotation ordinale ou cardinale, c'est-à-dire qu'ils n'impliquent ici aucune notion d'ordre, de quantité ou une quelconque catégorisation des éléments ou composants auxquels ces termes sont attachés. Ils n'ont en l'espèce pour vocation que de faciliter la compréhension de l'invention en permettant de différencier entre elles certaines caractéristiques techniques que peut présenter le dispositif d'arthrodèse selon l'invention, ainsi que les différentes étapes que peut avantageusement comprendre la méthode chirurgicale visant avantageusement la mise en oeuvre dudit dispositif d'arthrodèse.

Egalement, les termes « *postérieur* », « *antérieur* », « *médial* » et « *latéral* » sont préférentiellement utilisés dans la présente description pour qualifier des éléments ou des caractéristiques en lien avec leur orientation respective relativement au corps du patient, en usage normal du dispositif d'arthrodèse selon l'invention, et en particulier du clou 1 de ce dernier. Ainsi, le terme « *médial* » est préférentiellement utilisé pour désigner un élément ou composant du dispositif d'arthrodèse qui destiné à être positionné et orienté du côté le plus proche de l'axe medio-sagittal (ou axe médian) du corps du patient, autrement dit le côté orienté vers l'intérieur de la cheville et de la jambe du patient. Par opposition, le terme « latéral » est utilisé en relation avec le côté le plus éloigné de l'axe medio-sagittal. Suivant la même logique, les termes « *postérieur* » et « *antérieur* » renvoient de préférence respectivement à un positionnement vers l'arrière, respectivement vers l'avant, relativement au plan frontal du patient.

### POSSIBILITE D'APPLICATION INDUSTRIELLE

L'invention trouve son application dans la conception et la fabrication de dispositifs destinés à être utilisés pour réaliser une arthrodèse et, plus spécifiquement, dans la conception et la fabrication de dispositifs destinés à être utilisés pour réaliser une arthrodèse de l'articulation d'une cheville, en particulier dans le cadre d'un traitement orthopédique.

## Revendications

1. - Dispositif d'arthrodèse de cheville comprenant un clou (1) destiné à être implanté dans un tibia (Ti), un talus (Ta) et un calcanéus (Ca) d'un patient, ledit clou (1) comprenant :
- une partie proximale (2) qui s'étend généralement selon une première direction d'extension (A-A') longitudinale, et
- une partie distale (4) qui prolonge ladite partie proximale (2) et s'étend généralement selon une deuxième direction d'extension (B-B') longitudinale sécante à ladite première direction d'extension (A-A') longitudinale, ladite partie distale (4) étant pourvue d'un premier orifice de fixation (8A),
**caractérisé en ce que** ledit dispositif comprend un premier moyen de fixation (9A) compressif, du type vis compressive (9A), et **en ce que** ledit clou (1) est conçu et configuré de sorte que, lorsque ledit clou (1) est implanté avec ladite partie distale (4) orientée vers l'arrière de la cheville du patient, ledit premier orifice de fixation (8A) est agencé pour recevoir et guider ledit premier moyen de fixation (9A) selon une première direction de fixation (F1-F1') qui s'étend à travers lesdits talus (Ta) et calcanéus (Ca) pour fixer ledit clou audit talus (Ta) et audit calcanéus (Ca), ledit premier orifice de fixation (8A) étant oblong selon ladite deuxième direction d'extension (B-B') longitudinale.

2. - Dispositif selon la revendication précédente, dans lequel lesdites première (A-A') et deuxième (B-B') directions d'extension sont sécantes selon un premier angle (θ₁) compris entre 5 et 10°, et de préférence égal à 7°.

3. - Dispositif selon l'une quelconque des revendications précédentes, dans lequel lesdites premières (A-A') et deuxième (B-B') directions d'extension sont inscrites dans un même plan principal (P), ledit clou (1) étant préférentiellement configuré et dimensionné de sorte que, lorsque ledit clou (1) est implanté, ledit plan principal (P) est sensiblement parallèle au plan sagittal du patient.

4. - Dispositif selon la revendication 3, dans lequel ladite première direction de fixation (F₁-F₁') est sécante à ladite première direction d'extension (A-A') selon un deuxième angle (θ₂) égal à 45° ± 3°, et sécante audit plan principal (P) selon un troisième angle (θ₃) égal à 9° ± 2°.

5. - Dispositif selon l'une quelconque des revendications précédentes, dans lequel ladite partie distale (4) est pourvue d'un deuxième orifice de fixation (8B), qui est configuré pour recevoir et guider un deuxième moyen de fixation (9B) selon une deuxième direction de fixation (F₂-F₂') pour fixer ledit clou (1) au seul dit calcanéus (Ca).

6. - Dispositif selon les revendications 5 et 3, dans lequel ladite deuxième direction de fixation (F₂-F₂') est sécante à ladite première direction (A-A') selon un quatrième angle (θ₄) égal à 78° ± 3°, et sécante audit plan principal (P) selon un cinquième angle (θ₅) égal à 8° ± 2°.

7. - Dispositif selon l'une quelconque des revendications précédentes, dans lequel ladite partie distale (4) est pourvue d'un troisième orifice de fixation (8C), qui est configuré pour recevoir et guider un troisième moyen de fixation (9C) selon une troisième direction de fixation (F₃-F₃') pour fixer ledit clou (1) au seul dit talus (Ta).

8. - Dispositif selon les revendications 7 et 3, dans lequel ladite troisième direction de fixation (F₃-F₃') est orthogonale audit plan principal (P).

9. - Dispositif selon l'une quelconque des revendications précédentes, dans lequel ladite partie proximale (2) est pourvue d'un quatrième orifice de fixation (8D), qui est configuré pour recevoir et guider un quatrième moyen de fixation (9D) selon une quatrième direction de fixation (F₄-F₄') pour fixer ledit clou (1) audit tibia (Ti).

10. - Dispositif selon les revendications 9 et 3, dans lequel ladite quatrième direction de fixation (F₄-F₄') est orthogonale audit plan principal (P).

11. - Dispositif selon la revendication 9 ou 10, dans lequel ladite partie proximale (2) est pourvue d'un cinquième orifice de fixation (8E), qui est configuré pour recevoir et guider un cinquième moyen de fixation (9E) selon une cinquième direction de fixation (F₅-F₅'), qui est de préférence parallèle à ladite quatrième direction de fixation (F₄-F₄'), pour fixer ledit clou (1) audit tibia (Ti).

12. - Dispositif selon la revendication précédente, dans lequel l'un desdits quatrième (8D) et cinquième (8E) orifices de fixation est de forme oblongue selon ladite première direction d'extension (A-A').

13. - Dispositif selon l'une quelconque des revendications précédentes, dans lequel ladite partie proximale (2) présente une forme générale cylindrique et est pourvue d'un moyen de blocage en rotation du clou (1) autour de ladite première direction d'extension (A-A'), ledit moyen de blocage comprenant de préférence au moins une rainure (6) ou ailette longitudinale.

14. - Dispositif selon l'une quelconque des revendications précédentes, lequel comprend en outre un instrument (10) de mise en place dudit clou (1) au sein de ladite articulation de la cheville du patient par voie plantaire, ledit instrument (10) étant préférentiellement conçu pour être accouplé de manière amovible à une extrémité distale (5) dudit clou (1).

15. - Dispositif selon la revendication précédente, dans lequel ledit instrument (10) de mise en place dudit clou (1) comprend un moyen de mise en compression (18) de l'articulation de la cheville du patient.

## Patentansprüche

1. Sprunggelenk-Arthrodesevorrichtung, umfassend einen Stift (1), der dazu bestimmt ist, in die Tibia (Ti), den Talus (Ta) und den Calcaneus (Ca) eines Patienten implantiert zu werden, wobei der genannte Stift (1) umfasst:
- einen proximalen Teil (2), der sich allgemein in einer ersten Längsausdehnungsrichtung (A-A') erstreckt, und
- einen distalen Teil (4), der den genannten proximalen Teil (2) verlängert und sich allgemein in einer zweiten Längsausdehnungsrichtung (B-B') erstreckt, welche die genannte erste Längsausdehnungsrichtung (A-A') schneidet, wobei der genannte distale Teil (4) mit einer ersten Befestigungsöffnung (8A) versehen ist,
**dadurch gekennzeichnet, dass** die genannte Vorrichtung ein erstes Kompressionsbefestigungsmittel (9A), vom Typ einer Kompressionsschraube (9A), umfasst, und dadurch, dass der genannte Stift (1) mit dem genannten distalen Teil (4) zur Rückseite des Sprunggelenks des Patienten gerichtet implantiert wird, die genannte erste Befestigungsöffnung (8A) angeordnet ist, um das genannte erste Befestigungsmittel (9A) in einer ersten Befestigungsrichtung (F1-F1`) aufzunehmen und zu führen, die sich durch den genannten Talus (Ta) und Calcaneus (Ca) erstreckt, um den genannten Stift an dem genannten Talus (Ta) und an dem genannten Calcaneus (Ca) zu befestigen, wobei der ersten Befestigungsöffnung (8A) länglich in der genannten zweiten Längsausdehnungsrichtung (B-B') ist.

2. Vorrichtung nach dem vorhergehenden Anspruch, wobei die genannte erste (A-A') und zweite (B-B') Ausdehnungsrichtung einander unter einem ersten Winkel (θ₁) zwischen 5 und 10°, und vorzugsweise gleich 7°, schneiden.

3. Vorrichtung nach einem der vorhergehenden Ansprüche, wobei die genannte erste (A-A') und zweite (B-B') Ausdehnungsrichtung in derselben Hauptebene (P) eingeschrieben sind, wobei der genannte Stift (1) vorzugsweise derart ausgelegt und bemessen ist, dass, wenn der genannte Stift (1) implantiert ist, die genannte Hauptebene (P) im Wesentlichen parallel zur Sagittalebene des Patienten ist.

4. Vorrichtung nach Anspruch 3, wobei die genannte erste Befestigungsrichtung (F₁-F₁') die genannte erste Ausdehnungsrichtung (A-A') unter einem zweiten Winkel (θ₂) gleich 45° ± 3° schneidet, und die genannte Hauptebene (P) unter einem dritten Winkel (θ₃) gleich 9° ± 2° schneidet.

5. Vorrichtung nach einem der vorhergehenden Ansprüche, wobei der genannte distale Teil (4) mit einer zweiten Befestigungsöffnung (8B) versehen ist, die dafür ausgelegt ist, um ein zweites Befestigungsmittel (9B) in einer zweiten Befestigungsrichtung (F₂-F₂') aufzunehmen und zu führen, um den genannten Stift (1) nur an dem genannten Calcaneus (Ca) zu befestigen.

6. Vorrichtung nach den Ansprüchen 5 und 3, wobei die genannte zweite Befestigungsrichtung (F₂-F₂') die genannte erste Richtung (A-A') unter einem vierten Winkel (θ₄) gleich 78° ± 3° schneidet, und die genannte Hauptebene (P) unter einem fünften Winkel (θ₅) gleich 8° ± 2° schneidet.

7. Vorrichtung nach einem der vorhergehenden Ansprüche, wobei der genannte distale Teil (4) mit einer dritten Befestigungsöffnung (8C) versehen ist, die dafür ausgelegt ist, um ein drittes Befestigungsmittel (9C) in einer dritten Befestigungsrichtung (F₃-F₃') aufzunehmen und zu führen, um den genannten Stift (1) nur an dem genannten Talus (Ta) zu befestigen.

8. Vorrichtung nach einem der Ansprüche 7 und 3, wobei die genannte dritte Befestigungsrichtung (F₃-F₃') orthogonal zu der genannten Hauptebene (P) ist.

9. Vorrichtung nach einem der vorhergehenden Ansprüche, wobei der genannte proximale Teil (2) mit einer vierten Befestigungsöffnung (8D) versehen ist, die dafür ausgelegt ist, um ein viertes Befestigungsmittel (9D) in einer vierten Befestigungsrichtung (F₄-F₄') aufzunehmen und zu führen, um den genannten Stift (1) an der genannten Tibia (Ti) zu befestigen.

10. Vorrichtung nach Anspruch 9 und 3, wobei die genannte vierte Befestigungsrichtung (F₄-F₄') orthogonal zu der genannten Hauptebene (P) ist.

11. Vorrichtung nach Anspruch 9 oder 10, wobei der genannte proximale Teil (2) mit einer fünften Befestigungsöffnung (8E) versehen ist, die dafür ausgelegt ist, um ein fünftes Befestigungsmittel (9E) in einer fünften Befestigungsrichtung (F₅-F₅') aufzunehmen und zu führen, die vorzugsweise parallel zu der genannten vierten Befestigungsrichtung (F₄-F₄') ist, um den genannten Stift (1) an der genannten Tibia (Ti) zu befestigen.

12. Vorrichtung nach dem vorhergehenden Anspruch, wobei eine von der genannten vierten (8D) und fünften (8E) Befestigungsöffnung eine längliche Form in der genannten ersten Ausdehnungsrichtung (A-A') aufweist.

13. Vorrichtung nach einem der vorhergehenden Ansprüche, wobei der genannte proximale Teil (2) eine allgemein zylindrische Form aufweist und mit einem Mittel zum Blockieren des Stifts (1) in Drehung um die erste Ausdehnungsrichtung (A-A') versehen ist, wobei das genannte Blockierungsmittel vorzugsweise mindestens eine Längsnut (6) oder - rippe umfasst.

14. Vorrichtung nach einem der vorhergehenden Ansprüche, welche ferner ein Instrument (10) zur Platzierung des genannten Stifts (1) im Inneren der genannten Gelenkverbindung des Sprunggelenks des Patienten auf plantarem Weg umfasst, wobei das genannte Instrument (10) vorzugsweise ausgebildet ist, um lösbar mit einem distalen Ende (5) des genannten Stifts (1) gekoppelt zu werden.

15. Vorrichtung nach dem vorhergehenden Anspruch, wobei das genannte Instrument (10) zum Platzieren des genannten Stifts (1) ein Mittel (18) zum Komprimieren der Gelenkverbindung des Sprunggelenks des Patienten umfasst.

## Claims

1. An ankle arthrodesis device comprising a nail (1) intended to be implanted in a tibia (Ti), a talus (Ta) and a calcaneus (Ca) of a patient, said nail (1) comprising:
- a proximal portion (2) which generally extends according to a first longitudinal direction of extension (A-A'), and
- a distal portion (4) which prolongs said proximal portion (2) and generally extends according to a second longitudinal direction of extension (B-B') secant to said first longitudinal direction of extension (A-A'), said distal portion (4) being provided with a first fastening orifice (8A),
**characterized in that** said distal portion (4) said device comprises a first compressive fastening means (9A), of compressive screw (9A) type, and **in that** said nail (1) is designed and configured so that, when said nail (1) is implanted with said distal portion (4) directed rearwards of the ankle of the patient, said first fastening orifice (8A) is arranged so as to receive and guide said first fastening means (9A) according to a first fastening direction (F₁-F_{1'}) which extends through said talus (Ta) and calcaneus (Ca) so as to fasten said nail to said talus (Ta) and to said calcaneus (Ca), said first fastening orifice (8A) being oblong according to said second longitudinal direction of extension (B-B').

2. The device according to the preceding claim, wherein said first (A-A') and second (B-B') directions of extension are secant according to a first angle (θ₁) comprised between 5 and 10°, and preferably equal to 7°.

3. The device according to any one of the preceding claims, wherein said first (A-A') and second (B-B') directions of extension are inscribed within a same main plane (P), said nail (1) being preferably configured and dimensioned so that, when said nail (1) is implanted, said main plane (P) is substantially parallel to the sagittal plane of the patient.

4. The device according to claim 3, wherein said first fastening direction (F₁-F_{1'}) is secant to said first direction of extension (A-A') according to a second angle (θ₂) equal to 45°±3°, and secant to said main plane (P) according to a third angle (θ₃) equal to 9°±2°.

5. The device according to any one of the preceding claims, wherein said distal portion (4) is provided with a second fastening orifice (8B), which is configured so as to receive and guide a second fastening means (9B) according to a second fastening direction (F₂-F_{2'}) in order to fasten said nail (1) only to said calcaneus (Ca).

6. The device according to claims 5 and 3, wherein said second fastening direction (F₂-F_{2'}) is secant to said first direction (A-A') according to a fourth angle (θ₄) equal to 78°±3°, and secant to said main plane (P) according to a fifth angle (θ₅) equal to 8°±2°.

7. The device according to any one of the preceding claims, wherein said distal portion (4) is provided with a third fastening orifice (8C), which is configured so as to receive and guide a third fastening means (9C) according to a third fastening direction (F₃-F_{3'}) in order to fasten said nail (1) only to said talus (Ta).

8. The device according to claims 7 and 3, wherein said third fastening direction (F₃-F_{3'}) is orthogonal to said main plane (P).

9. The device according to any one of the preceding claims, wherein said proximal portion (2) is provided with a fourth fastening orifice (8D), which is configured so as to receive and guide a fourth fastening means (9D) according to a fourth fastening direction (F₄-F_{4'}) in order to fasten said nail (1) to said tibia (Ti).

10. The device according to claims 9 and 3, wherein said fourth fastening direction (F₄-F_{4'}) is orthogonal to said main plane (P).

11. The device according to claim 9 or 10, wherein said proximal portion (2) is provided with a fifth fastening orifice (8E), which is configured so as to receive and guide a fifth fastening means (9E) according to a fifth fastening direction (F₅-F_{5'}), which is preferably parallel to said fourth fastening direction (F₄-F₄'), in order to fasten said nail (1) to said tibia (Ti).

12. The device according to the preceding claim, wherein one amongst said fourth (8D) and fifth (8E) fastening orifices has an oblong shape according to said first direction of extension (A-A').

13. The device according to any one of the preceding claims, wherein said proximal portion (2) has a cylindrical general shape and is provided with a means for blocking the rotation of the nail (1) about said first direction of extension (A-A'), said blocking means preferably comprising at least one longitudinal groove (6) or fin.

14. The device according to any one of the preceding claims, which further comprises an instrument (10) for setting said nail (1) in place within said joint of the ankle of the patient through a plantar approach, said instrument (10) being preferably designed so as to be removably coupled to a distal end (5) of said nail (1).

15. The device according to the preceding claim, wherein said instrument (10) for setting said nail (1) in place comprises a means (18) for compressing the joint of the ankle of the patient.
